# EUROPEAN PATENT APPLICATION

(11) **EP 4 696 707 A1**
(43) Date of publication of application: **18.02.2026**
(21) Application number: 24789102.1
(22) Date of filing: 15.04.2024
(51) Int. Cl.: C07K 14/47, A61K 38/00

(54) **SINGLE FUSION PROTEIN AND PHARMACEUTICAL COMPOSITION COMPRISING SAME**

(30) Priority: 13.04.2023 KR 20230048655
(71) Applicant: Medi&Gene Inc., Seoul 02841 (KR); Korea University Research and Business Foundation, Seoul 02841 (KR)
(72) Inventor: SHIN, Min Jeong, Seoul 06285 (KR); JEONG, In Hyeok, Goyang-si Seoul 10521 (KR); LEE, Se Hee, Seongnam-si Gyeonggi-do 13517 (KR)
(74) Representative: Reddie & Grose LLP
(86) International application number: PCT/KR2024/005010
(87) International publication number: WO 2024/215162

(57) **Abstract**

The present invention relates to fusion proteins comprising IF1 protein or a peptide derived therefrom and an Fc region of an immunoglobulin, and a pharmaceutical composition comprising the same. The fusion protein according to the present invention exhibits improved pharmacological efficacy, in vivo persistence and stability of proteins, and the pharmaceutical composition comprising the fusion protein as an active ingredient can be usefully used as a therapeutic agent for cancer, neurological diseases, diabetes, obesity diseases, dyslipidemia, metabolic diseases, nonalcoholic fatty liver diseases, nonalcoholic steatohepatitis, hair loss prevention or hair growth promotion, sarcopenia, and obese sarcopenia.

## Description

### [TECHNICAL FIELD]

The present invention relates to fusion proteins comprising IF1 protein or a peptide derived therefrom and an Fc region of an immunoglobulin, and a pharmaceutical composition comprising the same, and the pharmaceutical composition can be usefully used for therapeutic uses of treating cancer, neurological diseases, diabetes, obesity diseases, dyslipidemia, metabolic diseases, nonalcoholic fatty liver diseases, nonalcoholic steatohepatitis, hair loss prevention or hair growth promotion, sarcopenia, and obese sarcopenia.

### [BACKGROUND ART]

Protein pharmaceuticals are pharmaceuticals composed of amino acids, such as insulin, growth factors, and antibodies, and can be classified into the first generation, which utilizes structures identical to natural proteins, and the second generation, which is a sustained-release formulation in which efficacy is improved by applying protein engineering technology or half-life is prolonged by conjugating an auxiliary substance.

Since protein pharmaceuticals are manufactured using biologically derived substances, they exhibit lower inherent toxicity and clear mechanisms of action, thereby having an advantage of superior therapeutic effects compared to chemically synthesized pharmaceuticals. However, since they are high molecular weight substances having high order structures, there is a disadvantage of lower physicochemical stability compared to low molecular weight compounds, and accordingly, a shorter half-life in vivo. In addition, because they are produced in living organisms, the types and degrees of modifications bound to the proteins vary depending on the types of living organisms or the culture conditions, and thus, there is a problem that it is difficult to secure homogeneity.

Especially, since the intrinsic activity of a protein is altered when its three-dimensional structure is modified by substitution, addition, or the like, of amino acids, it has still remained difficult to increase the half-life, or to enhance the intrinsic activity itself, while maintaining the intrinsic activity itself.

### [DISCLOSURE]

### [TECHNICAL PROBLEM]

In the foregoing circumstances, the present inventors have conducted research to increase the in vivo activity and half-life of peptides, and have found that, when an Fc sequence was conjugated to the peptide, the half-life was prolonged and the activity of the peptide itself was also enhanced, thereby completing the present invention.

Accordingly, an object of the present invention is to provide a fusion protein having the following structure and a pharmaceutical composition comprising the same:
an amino acid sequence selected from the group consisting of SEQ ID NOs: 8 to 10 and 46;
a linker connected to the C-terminus of the amino acid sequence; and
an Fc sequence connected to a terminus of the linker.

### [TECHNICAL SOLUTION]

In order to achieve the above object, one aspect of the present invention provides the following fusion protein:
an amino acid sequence selected from the group consisting of SEQ ID NOs: 8 to 10 and 46;
a linker connected to the C-terminus of the amino acid sequence; and
an Fc sequence connected to a terminus of the linker.

The amino acid sequence consisting of SEQ ID NO: 8 is a wild-type ATPIF1 protein, and the sequences of SEQ ID NOs: 9, 10 and 46 are sequences derived from the wild-type ATPIF1 protein. The sequence of SEQ ID NO: 9 consists of the 1 to 60th amino acid sequence of the wild-type ATPIF1 protein, and the sequence of SEQ ID NO: 10 consists of the 1 to 47th amino acid sequence of the wild-type ATPIF1 protein. The sequence of SEQ ID NO: 46 is one in which G20S, F34M mutations are introduced into the sequence of SEQ ID NO: 9.

ATPIF1 (ATPase inhibitory factor 1) is a major protein that is expressed inside cells, binds to ATPase present in the mitochondrial membrane, interferes with rotary motion, and consequently inhibits ATP synthesis/hydrolysis by the electron transport chain, thereby affecting intracellular energy regulation and mitochondrial homeostasis.

According to one embodiment of the present invention, the linker sequence may be a sequence of SEQ ID NO: 40 or SEQ ID NO: 41, but not limited thereto, and any one used as a peptide linker in the art to which the present invention pertains may be used without limitation.

According to one embodiment of the present invention, the Fc sequence may be a sequence of SEQ ID NO: 42 or SEQ ID NO: 43, but not limited thereto, any one used as an Fc sequence in the art to which the present invention pertains may be used without limitation. The Fc sequence may be an Fc sequence of an immunoglobulin (IgG1).

The sequence of SEQ ID NO: 43 is a sequence in which a mutation is introduced in the sequence of SEQ ID NO: 42, in order to prevent unnecessary immune responses from occurring when an Fc fusion substance functions as a drug, by effectively suppressing an effector function (inducing immune responses of antibodies) resulting from Fc conjugation.

According to one embodiment of the present invention, in the amino acid sequence selected from the group consisting of SEQ ID NOs: 8 to 10 and 46, one or more amino acids selected from the group consisting of the 8th, 9th, 10th, 11th, 13th, 14th, 15th, 16th, 17th, 22th, 23th, 24th, 25th, 26th, 29th, 30th, 31th, 32th, 33th, 34th and 31th amino acids may be substituted with alanine. The amino acids at the aforementioned positions did not show a significant difference in binding ability to ATP5B protein even when substituted with alanine. This result indicates that the structure of the protein is not significantly altered by alanine substitution, and indicates that the in vivo activity of the amino acid sequence selected from the group consisting of SEQ ID NOs: 8 to 10 and 46 is also maintained.

According to one embodiment of the present invention, the fusion protein may consist of a sequence selected from the group consisting of SEQ ID NOs: 1 to 3, 13, 14 and 15, but not limited thereto.

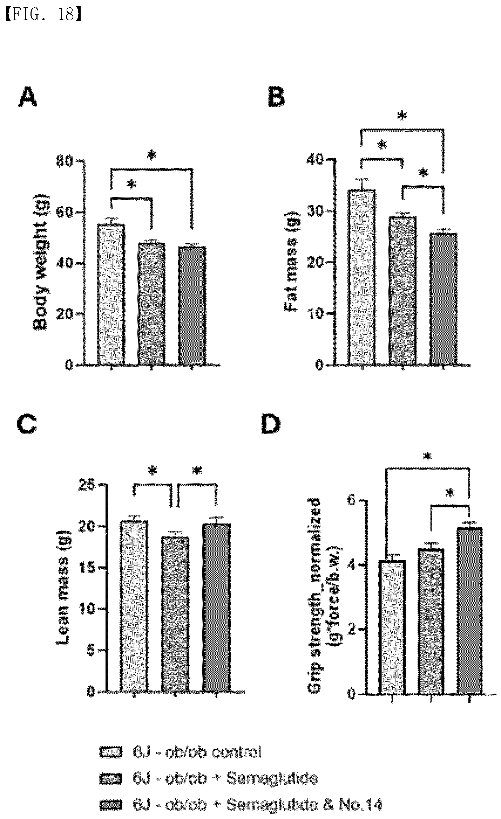

According to one embodiment of the present invention, the fusion protein may comprise an additional sequence and consist of the following structure, and may consist of the sequence of SEQ ID NO: 7;
an amino acid selected from the group consisting of SEQ ID NOs: 8 to 10 and 46;
a linker connected to the C-terminus of the amino acid sequence;
a GLP-1 (Glucagon like peptide-1) sequence consisting of SEQ ID NO: 44 connected to a terminus of the linker;
a GLP-1 linker consisting of SEQ ID NO: 45 connected to a terminus of the GLP-1 sequence; and
the Fc sequence connected to a terminus of the GLP-1 linker showed more significant changes (decrease in the amount of adipose tissue/increase in the amount of muscle tissue), compared to a group administered with semaglutide alone, when administered to obesity-induced mice (FIGs. 18B, C).

On the other hand, the inventors of the present invention have confirmed various in vivo activities of the fusion protein.

Accordingly, another aspect of the present invention provides a pharmaceutical composition for treating or treating cancer comprising the fusion protein as an active ingredient.

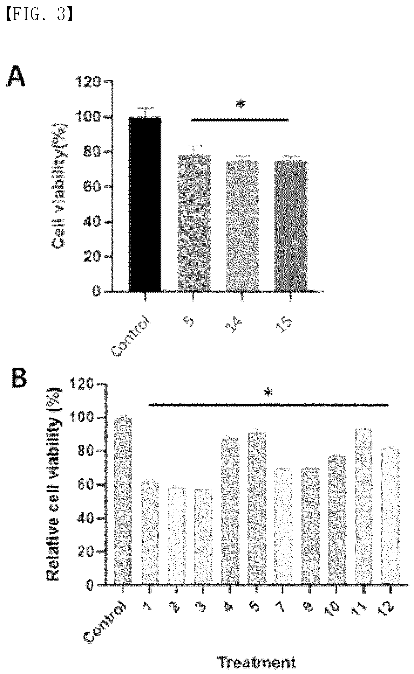

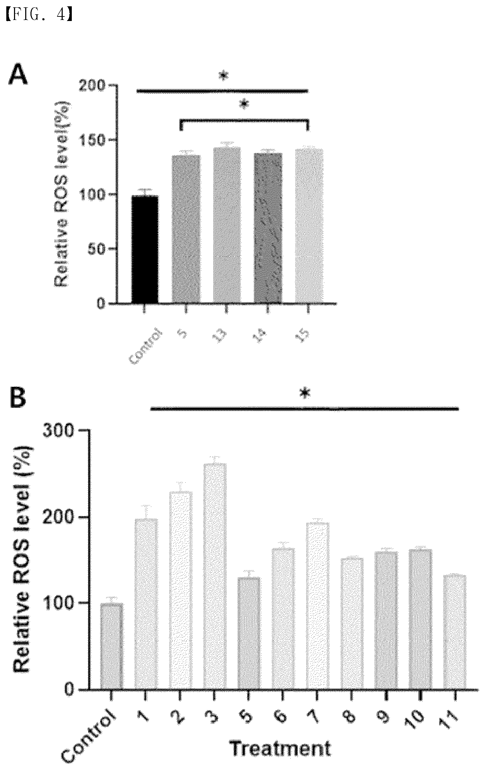

According to one embodiment of the present invention, the fusion proteins of SEQ ID NOs: 1 to 3, 7 and 13 to 15 have excellent anticancer activity, since they effectively kill cancer cells, and promote generation of reactive oxygen (FIG. 3 and FIG. 4).

In the present invention, the cancer may be a solid cancer, and specifically, may be selected from the group consisting of lung cancer, ovarian cancer, colorectal cancer, colon cancer, pancreatic cancer, liver cancer, cervical cancer, kidney cancer, stomach cancer, prostate cancer, breast cancer, brain tumor, uterine cancer and bladder cancer, but is not limited thereto.

The term used in the present invention, "prevention" means any action of suppressing the progression or delaying the onset of a disease by administration of the pharmaceutical composition according to the present invention.

The term used in the present invention, "treatment" means any action of improving or favorably changing symptoms of a disease by administration of the pharmaceutical composition according to the present invention.

In addition, the present invention provides a pharmaceutical composition for preventing or treating metabolic syndrome comprising the fusion protein as an active ingredient.

The metabolic syndrome refers to a phenomenon in which various metabolism-related diseases such as arteriosclerosis and hypertension, obesity, diabetes, hyperlipidemia and the like occur simultaneously. Accordingly, the metabolic syndrome includes diseases such as obesity, diabetes, dyslipidemia, and the like.

According to one embodiment of the present invention, the fusion protein can be usefully used for a use of treating metabolic syndrome, since it inhibits fat accumulation (Example 3-3), improves impaired glucose tolerance (Example 4-5) and improves obesity (Example 4-3).

The fusion protein of the present invention also has an excellent effect of improving muscle loss (Example 4-1), and thus, it can be utilized as a pharmaceutical composition for preventing or treating diseases associated with muscle loss.

The diseases associated with muscle loss may be selected from the group consisting of sarcopenia, Duchenne muscular dystrophy, muscular atrophy, myasthenia, muscular dystrophy, myotonia, hypotonia and muscular weakness.

In addition, other aspect of the present invention provides a pharmaceutical composition for preventing or treating nonalcoholic fatty acid comprising the fusion protein as an active ingredient.

According to one embodiment of the present invention, the fusion protein has a significantly excellent effect of improving nonalcoholic fatty acid by reducing liver damage, blood cholesterol, and triglycerides in an animal model induced with nonalcoholic fatty liver (Example 4-4).

In the present specification, nonalcoholic fatty liver disease (NAFLD) refers to a state in which fat is accumulated in hepatocytes despite no alcohol consumption or only light alcohol consumption. Rather than a single disease, it includes various types of liver diseases ranging from simple steatosis without accompanying inflammation to steatohepatitis (nonalcoholic steatohepatitis, NASH), cirrhosis (hepatocirrhosis).

On the other hand, in recent years, instead of the term nonalcoholic fatty liver disease, steatohepatitis occurring due to abnormalities in metabolic processes such as obesity and diabetes has been referred to as metabolic dysfunction-associated steatohepatitis (MASH).

Other aspect of the present invention provides a pharmaceutical composition for preventing hair loss or promoting hair growth comprising the fusion protein as an active ingredient.

According to one embodiment of the present invention, the fusion protein can be usefully used for a use of preventing/improving hair loss and promoting hair growth, as it promotes survival and proliferation of follicular cells (Example 3-5).

Other aspect of the present invention provides a pharmaceutical composition for protecting brain neuron cells comprising the fusion protein as an active ingredient.

According to one embodiment of the present invention, the fusion protein can be usefully used for a use of protecting brain neuron cells, since it has an excellent effect of inhibiting brain cell death from a neurotoxin substance, rotenone (Example 3-4).

Meanwhile, the inventors of the present invention have also confirmed in vivo activity of the sequence consisting of SEQ ID NO: 10, the sequence consisting of SEQ ID NO: 11 and the sequence consisting of SEQ ID NO: 12, in addition to the fusion protein.

Therefore, other aspect of the present invention provides a pharmaceutical composition for preventing or treating cancer, preventing or treating muscle diseases associated with muscle loss, preventing or treating metabolic syndrome, preventing or treating nonalcoholic fatty liver diseases, preventing muscle loss or promoting hair growth and protecting brain neuron cells, comprising a sequence selected from SEQ ID NOs: 10, 11 and 12 as an active ingredient.

The pharmaceutical composition according to one embodiment of the present invention may be applied to all animals including humans, dogs, chickens, pigs, cattle, sheep, guinea pigs, or monkeys.

The pharmaceutical composition according to one embodiment of the present invention may comprise additives such as diluents, excipients, lubricants, binders, disintegrants, buffers, dispersants, surfactants, coloring agents, flavoring agents, or sweeteners, as necessary. The pharmaceutical composition according to one embodiment of the present invention may be prepared by a conventional method in the art.

In the present invention, the carriers, excipients, and diluents that may be included in the pharmaceutical composition may include lactose, dextrose, sucrose, sorbitol, mannitol, xylitol, erythritol, maltitol, starch, acacia gum, alginate, gelatin, calcium phosphate, calcium silicate, cellulose, methyl cellulose, microcrystalline cellulose, polyvinylpyrrolidone, water, methyl hydroxybenzoate, propyl hydroxybenzoate, talc, magnesium stearate, and mineral oil.

The pharmaceutical composition according to one embodiment of the present invention may be administered orally, rectally, percutaneously, intravenously, intramuscularly, intraperitoneally, intramedullary, intradurally, or subcutaneously.

Formulations for oral administration may be tablets, pills, soft or hard capsules, granules, powders, liquids, or suspensions, but are not limited thereto. Formulations for parenteral administration may be injections, drops, gels, suspensions, emulsions, suppositories, patches, or sprays, but are not limited thereto.

The pharmaceutical composition may be in the form of an aqueous or oily suspension for sterile injection. The suspension may be formulated using suitable dispersants or wetting agents (e.g., Tween 80) and suspension agents, according to techniques known in the art. Preparations for sterile injection may also be a sterile injection solution or suspension in a nontoxic parenterally acceptable diluent or solvent (e.g., solution in 1,3-butanediol). Vehicles and solvents that can be acceptably used include mannitol, water, Ringer' s solution, and isotonic sodium chloride solution. In addition, sterile non-volatile oils are commonly used as a solvent or suspension medium. For this purpose, any less-irritating non-volatile oil, including synthetic mono- or diglycerides, may be used. Fatty acids such as oleic acid and glyceride derivatives thereof, as same as pharmaceutically acceptable natural oils (e.g., olive oil or castor oil), particularly polyoxyethylated forms thereof, are also useful in preparations for injection.

Parenteral administration of the pharmaceutical composition according to the present invention is particularly useful when the intended treatment is associated with a site or organ that is easily accessible by local application. Carriers for topical administration of the composition of the present invention include, but are not limited to, mineral oil, liquid paraffin, white Vaseline, propylene glycol, polyoxyethylene, polyoxypropylene compounds, emulsifying wax, and water.

The active ingredient of the pharmaceutical composition according to the present invention may vary depending on the age, gender, body weight, pathological condition and its severity of a subject to be administered, an administration route or judgement of a prescriber. Determination of the dosage based on such factors is within the level of a person skilled in the art, and its daily dose may be for example, 10 ng/kg/day to 10 mg/kg/day, specifically, 0.1 µg/kg/day to 1 mg/kg/day, more specifically, 1 µg/kg/day to 100 µg/kg/day, further more specifically, 2 µg/kg/day to 50 µg/kg/day, but when a difference in effect depending on the dosage is shown, this may be appropriately adjusted. The pharmaceutical composition according to one embodiment of the present invention may be administered once to 3 times a day, but is not limited thereto.

Other aspect of the present invention provides a health functional food composition for preventing or improving cancer, preventing or improving muscle diseases associated with muscle loss, preventing or improving metabolic syndrome, preventing or improving nonalcoholic fatty liver diseases, preventing muscle loss or promoting hair growth and protecting brain neuron cells, comprising the fusion protein as an active ingredient.

The term used in the present invention, "improvement" means any action of at least reducing parameters related to a state in which a disease is treated, for example, the degree of symptoms.

In the health functional food composition, the contents related to the fusion protein are the same as described above, and therefore, description for the overlapping matters is omitted.

The formulation of the food composition according to one embodiment of the present invention is not particularly limited, but for example, it may be formulated as a tablet, granule, powder, liquid, solid preparation, or the like. Each formulation may appropriately select and combine components commonly used in the corresponding art according to the formulation or intended use without difficulty by a person skilled in the art, and when applied together with other ingredients, a synergistic effect may occur.

The terms used in the present specification are intended only for the purpose of describing specific embodiments, and are not intended to limit the present invention. A term in which the number is omitted before a noun is not intended to limit the quantity, but indicates that one or more of the mentioned noun items are present. The terms "comprising," "having," and "containing" are to be interpreted as open terms (that is, meaning "including but not limited to" ).

The recitation of numerical ranges is merely a convenient way of individually mentioning each separate numerical value belonging to the range, and unless otherwise specified, each separate numerical value is incorporated into the present specification as if individually mentioned. All endpoints of ranges are included within the range and may be independently combined.

All methods mentioned in the present specification may be carried out in an appropriate order unless otherwise specified or clearly contradicted by the context. The use of any example and all examples, or exemplary language (for example, "such as" ), is unless included in the claims, only for better describing the present invention, and is not intended to limit the scope of the present invention. Any language in the specification should not be interpreted as essential to the implementation of the present invention with respect to any non-claimed component. Unless otherwise defined, technical and scientific terms used in the present specification have the same meaning as commonly understood by a person having ordinary skill in the art to which the present invention pertains.

### [ADVANTAGEOUS EFFECTS]

The fusion protein according to the present invention exhibits improved pharmacological efficacy, in vivo persistence and protein stability, and a pharmaceutical composition comprising the fusion protein as an active ingredient can be usefully used as a therapeutic agent for cancer, neurological diseases, diabetes, obesity diseases, dyslipidemia, metabolic diseases, nonalcoholic fatty liver diseases, nonalcoholic steatohepatitis, hair loss prevention or hair growth promotion, sarcopenia, and obese sarcopenia.

### [BRIEF DESCRIPTION OF THE DRAWINGS]

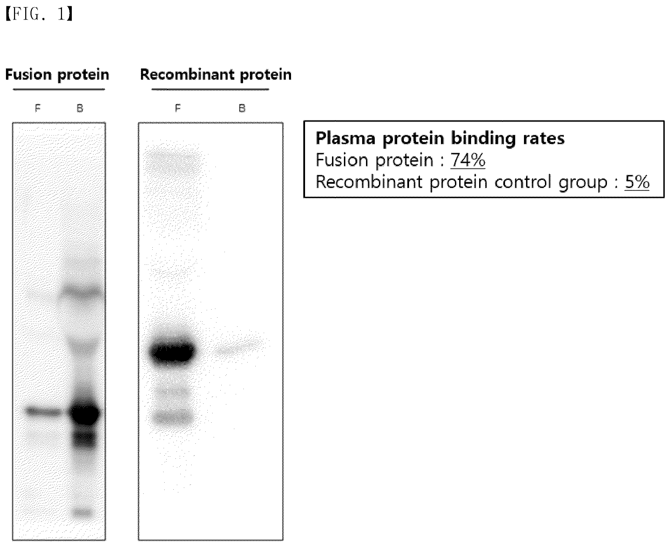

FIG. 1 shows the results of comparing the plasma protein binding rates of the fusion protein (SEQ ID NO: 13) or recombinant protein (SEQ ID NO: 5) of the present invention (F: Free, unbound free protein, B: Bound, bound protein).

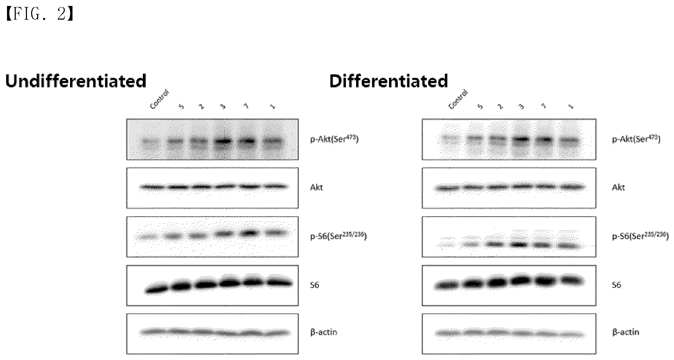

FIG. 2 shows the results of comparing the cell activity of the fusion protein (SEQ ID NO: 13) or recombinant protein (SEQ ID NO: 5) of the present invention through the phosphorylation levels of AKT and S6, which are markers of cell signaling pathway activation in C2C12 differentiated or undifferentiated muscle cells.
FIG. 3 shows the results of comparing the anticancer activity of the test substances (SEQ ID NOs: 1, 2, 3, 4, 7, 9, 10, 11, 12, 14, 15) or recombinant protein (SEQ ID NO: 5) of the present invention by the cell viability through MTT assay in the MBA-MB-231 breast cancer cell line (*p value<0.05 compared to control).
FIG. 4 shows the results of comparing the anticancer activity of the substances (SEQ ID NOs: 1, 2, 3, 6, 7, 8, 9, 10, 11, 13, 14, 15) or recombinant protein (SEQ ID NO: 5) of the present invention by the reactive oxygen species generation level through ROS assay in the MBA-MB-23 breast cancer cell line (*p value<0.05 compared to control).

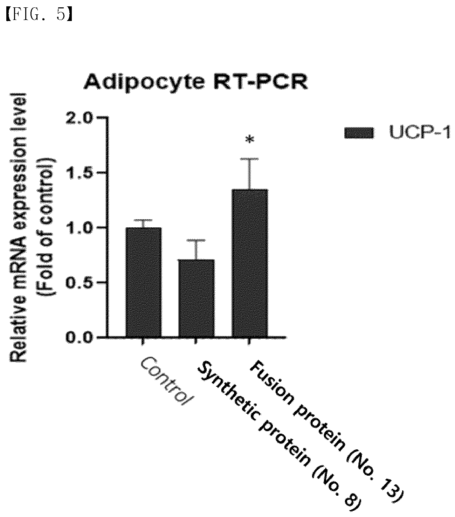

FIG. 5 shows the results of comparing the UCP-1 expression level through RT-PCR after treating the 3T3-L1 fat cell line with the fusion protein (SEQ ID NO: 13) or synthetic peptide (SEQ ID NO: 8) of the present invention (*p value<0.05 compared to control).

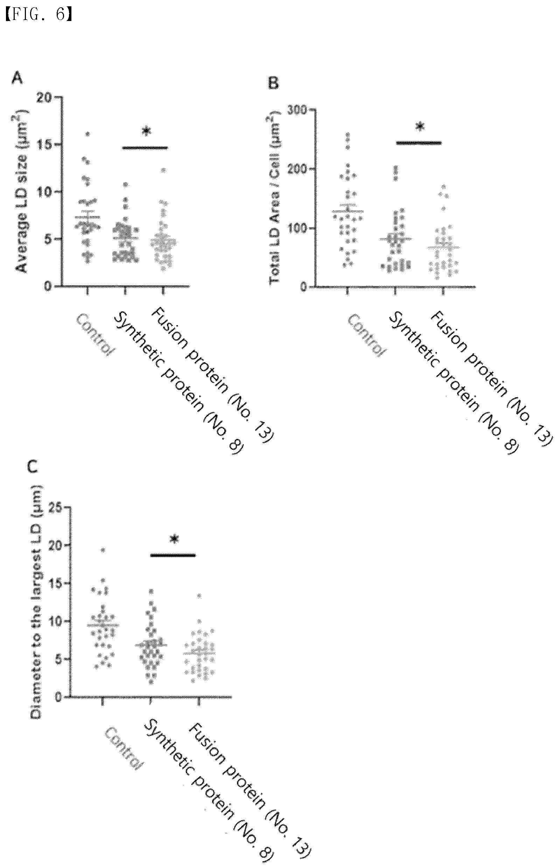

FIG. 6 shows the results of comparing (A) changes in lipid droplet size, (B) total lipid droplet area, and (C) the maximum diameter of each lipid droplet after treating the 3T3-L1 fat cell line with the fusion protein (SEQ ID NO: 13) or synthetic peptide (SEQ ID NO: 8) of the present invention (*p value<0.05 compared to control).

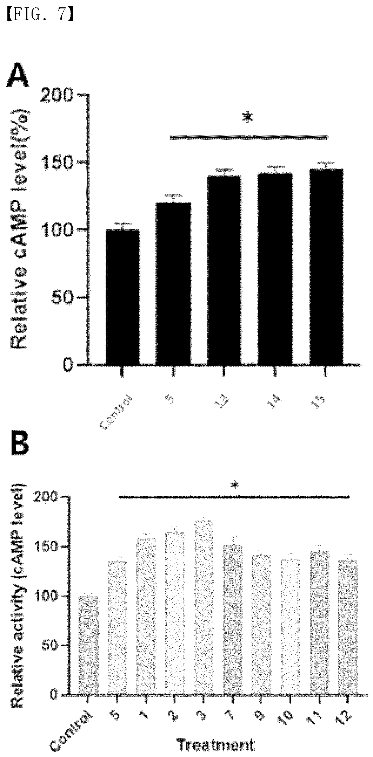

FIG. 7 shows the results of comparing the fat energy metabolic activity of the substances (SEQ ID NOs: 1, 2, 3, 7, 9, 10, 12, 13, 14, 15) of the present invention, as measured by cAMP assay, in the 3T3-L1 fat undifferentiated cells (*p value<0.05 compared to control).

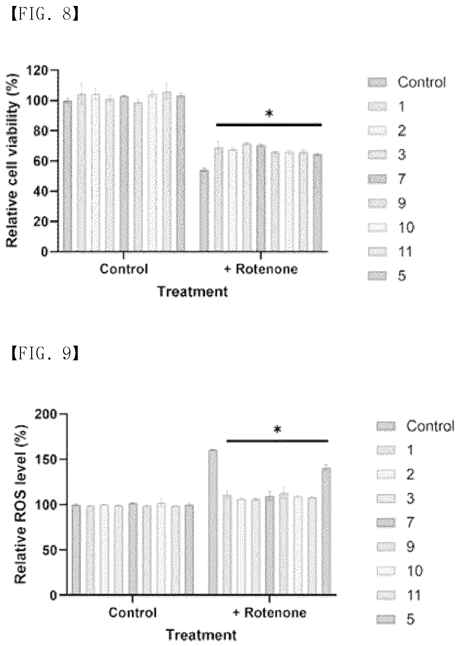

FIG. 8 shows the results of comparing the cell viability after treating the SH-SY5Y dopaminergic neuron cells with the substances (SEQ ID NOs: 1, 2, 3, 7, 9, 10, 11) of the present invention with rotenone, as measured by MTT assay (*p value<0.05 compared to control).
FIG. 9 shows the results of comparing the degree of inhibition of reactive oxygen generation after treating the SH-SY5Y dopaminergic neuron cells with the substances (SEQ ID NOs: 1, 2, 3, 7, 9, 10, 11) of the present invention with rotenone, as measured by ROS assay (*p value<0.05 compared to control).

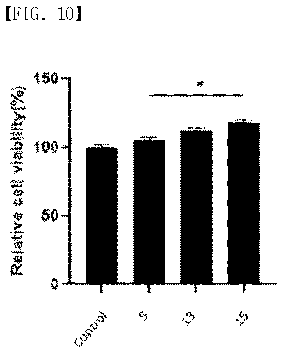

FIG. 10 shows the results of verifying the cellular effect by MTT assay after treating the follicular cells with the substances (SEQ ID NOs: 13, 15) of the present invention (*p value<0.05 compared to control).

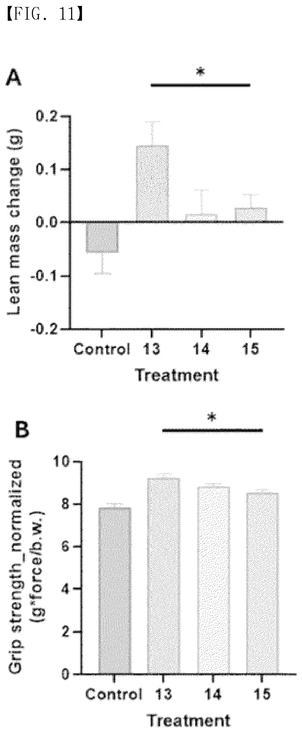

FIG. 11 shows the results of quantifying the in vivo functions of the substances (SEQ ID NOs: 13, 14, 15) of the present invention for muscle loss and muscle-related diseases by (A) changes in muscle tissue mass, and (B) grip strength measurements in the sciatic nerve transection mouse model and comparing them with the control group (*p value<0.05 compared to control).

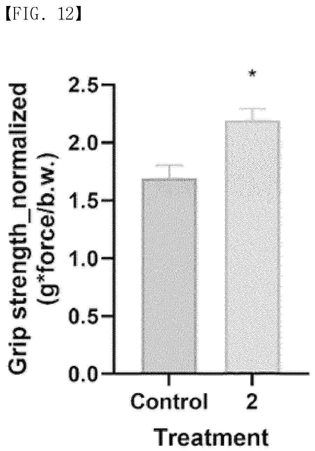

FIG. 12 shows the results of quantifying the in vivo functions of the substance (SEQ ID NO: 2) of the present invention for muscle loss and muscle-related diseases by the grip strength measurements in the hereditary muscular atrophy mouse model and comparing them with the control group (*p value<0.05 compared to control).

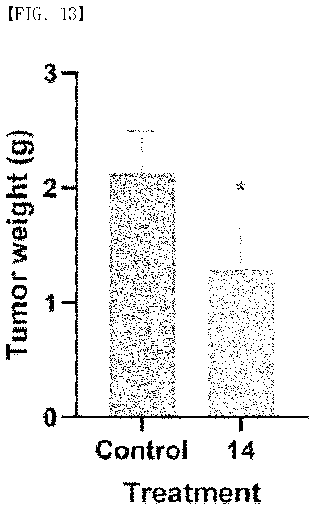

FIG. 13 shows the results of comparing the anticancer activity of the substance (SEQ ID NO: 14) of the present invention with the control group by quantifying the tumor growth size in the breast cancer xenograft mouse model (*p value<0.05 compared to control).

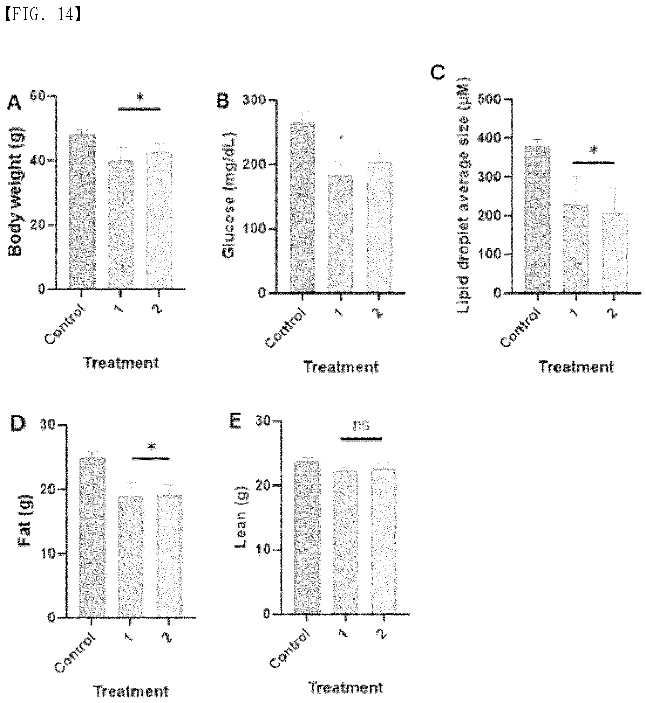

FIG. 14 shows the results of comparing the in vivo functions of the substances (SEQ ID NOs: 1, 2) of the present invention for obesity, lipid, glucose metabolism, and sarcopenic diseases with the control group by measuring (A) body weight, (B) blood glucose, (C) lipid droplet size in liver tissue, (D) fat mass, and (E) muscle mass in the diet-induced obesity-induced mouse model (ns, not significant, *p value<0.05 compared to control).

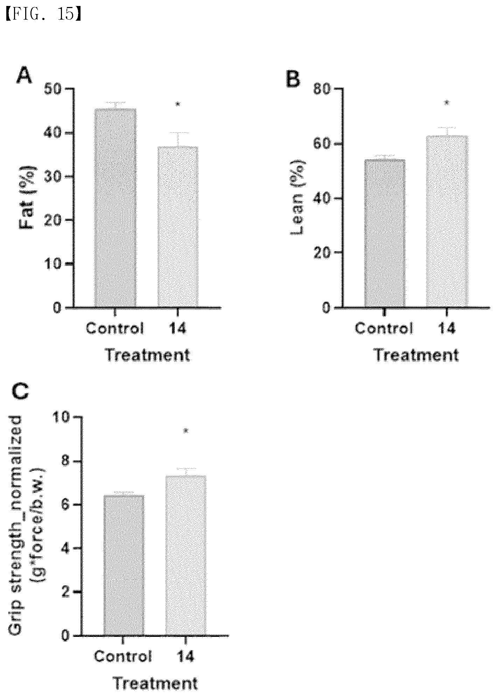

FIG. 15 shows the results of comparing the preventive in vivo functions of the substance (SEQ ID NO: 14) of the present invention for obesity, sarcopenic obesity and muscle disease with the control group by measuring (A) fat tissue ratio, (B) muscle tissue ration, and (C) grip strength in the diet-induced obesity-induced mouse model (*p value<0.05 compared to control).

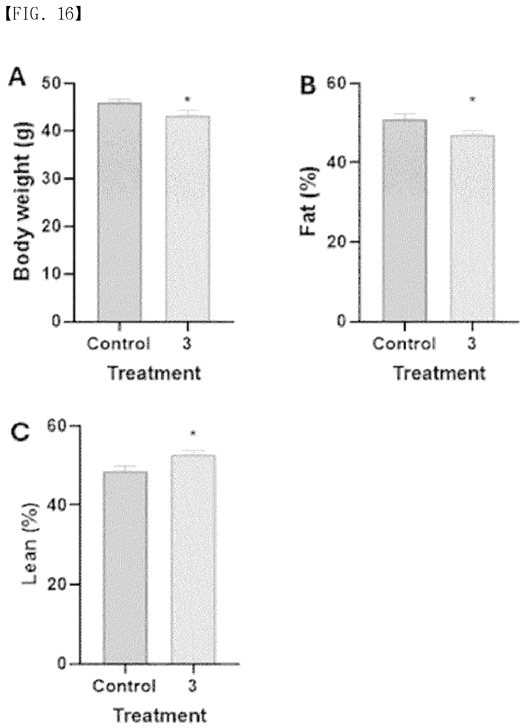

FIG. 16 shows the results of comparing the in vivo functions of the substance (SEQ ID NO: 3) of the present invention for obesity, sarcopenic obesity and muscle diseases with the control group by measuring (A) body weight, (B) fat tissue ratio, and (C) muscle tissue ratio in the diet-induced obesity-induced mouse model (*p value<0.05 compared to control).

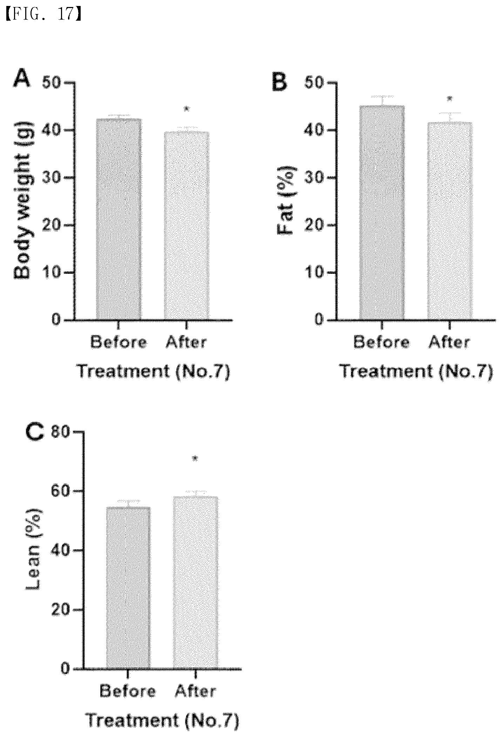

FIG. 17 shows the results of comparing the in vivo functions of the substance (SEQ ID NO: 7) of the present invention for obesity, sarcopenic obesity and muscle diseases by measuring (A) body weight, (B) fat tissue ration, and (C) muscle tissue ratio in the diet-induced obesity-induced mouse model, and comparing the data before and after treatment of the substance (*p value<0.05 compared to control).
FIG. 18 shows the results of comparing the in vivo functions of the substance (SEQ ID NO: 14) of the present invention for obesity, sarcopenic obesity and muscle diseases with the control group by measuring (A) body weight, (B) fat tissue ratio, (C) muscle tissue ratio, and (D) grip strength in the hereditary leptin-deficient mouse model (*p value<0.05 compared to each group).

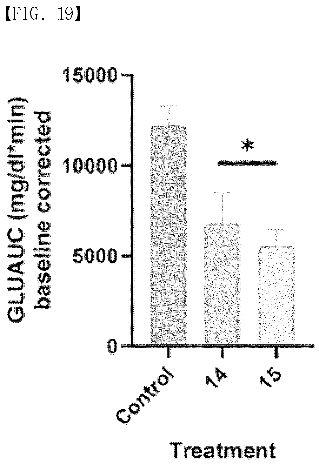

FIG. 19 shows the results of comparing the in vivo functions of the substances (SEQ ID NOs: 14, 15) of the present invention for obesity, sarcopenic obesity and glucose metabolic diseases with the control group by quantitative measurement of the glucose tolerance test in the diet-induced obesity-induced mice (*p value<0.05 compared to control).

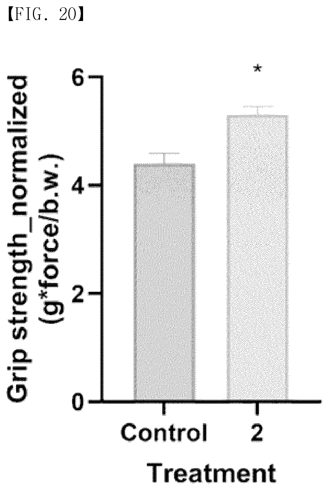

FIG. 20 shows the results of comparing the in vivo functions of the substance (SEQ ID NO: 2) of the present invention for glucose metabolic disease, muscle disease and complications thereof with the control group by quantitative measurement of the grip strength in the leptin receptor-deficient mouse model (*p value<0.05 compared to control).

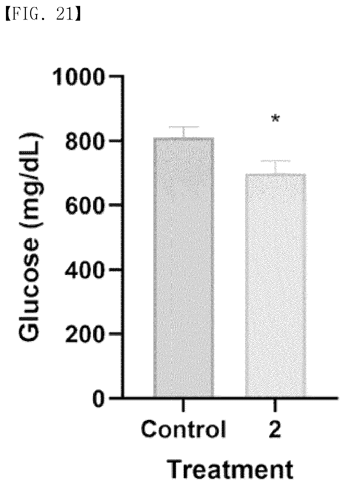

FIG. 21 shows the results of comparing the in vivo functions of the substance (SEQ ID NO: 2) of the present invention for glucose metabolic disease with the control group by measuring the blood glucose level in the leptin receptor-deficient mouse model (*p value<0.05 compared to control).

### [MODE FOR INVENTION]

Hereinafter, one or more embodiments will be described in more detail by examples. However, these examples are intended to exemplarily illustrate one or more embodiments only, but the scope of the present invention is not limited to these examples.

### Example 1: Preparation of transformed E. coli cells and production and verification of fusion proteins

In order to prepare fusion proteins of the wild-type ATPIF1 protein (SEQ ID NO: 8) and fragments thereof (SEQ ID NOs: 9, 10 and 12), vectors comprising sequences encoding the fusion proteins were transformed into an *E. coli* strain for expression, Shuffle to produce them. The transformed strain was cultured in LB medium under the condition of 37°C for 2 hours. After that, isopropyl-β-D-thiogalactopyranoside (IPTG) was added to the medium, followed by additional culture for 12 hours. After completing the culture, only the E. *coli* cells were collected, and the *E. coli* cells were separated into water-soluble fractions and insoluble fractions through the cell disruption (sonication) process and centrifugation. From each water-soluble fractions, through affinity-based resins, the expressed fusion proteins were purified: proteins of SEQ ID NOs: 1, 2, 3, 7, 13, 14 and 15: Protein A resin; proteins of SEQ ID NOs: 4 and 6: Sepharose resin).

In addition, peptides to be used in the present invention were synthesized by request to a specialized institution.

In Table 1 and Table 2 below, the description of the fusion proteins and peptides used in the present invention, and their sequences were summarized. The fusion protein and synthetic protein to be used as a control group were His tag Human ATPIF1 (SEQ ID NO: 5) and Human ATPIF1 (SEQ ID NO: 8).

**[Table 1]**

| SEQ ID NO: | Production method | Detailed description of substances |
|---|---|---|
| 1 | Recombinant protein | Shortened substance (1-60)-shortened linker-Fc fusion (mutant modified) |
| 2 | Recombinant protein | Shortened substance (1-60)-repeat-type linker-Fc fusion (mutant modified) |
| 3 | Recombinant protein | Shortened amino acid modified substance (1-60; G20S, F34M mutation introduced)-repeat-type linker-Fc fusion (mutant modified) |
| 4 | Recombinant protein | GST tag-wild-type substance (1-81) |
| 5 | Recombinant protein | His tag-wild-type substance (1-81) |
| 6 | Recombinant protein | GST tag-shortened substance (1-60) |
| 7 | Recombinant protein | Shortened substance (1-60)-linker-GLP1 sequence-GLP1-linker-Fc fusion |
| 8 | Synthetic peptide | Wild-type substance (1-81) |
| 9 | Synthetic peptide | Shortened substance (1-60) |
| 10 | Synthetic peptide | Shortened substance (1-47) |
| 11 | Synthetic peptide | Shortened amino acid modified substance (10-49; H49K mutation introduced) |
| 12 | Synthetic peptide | Shortened substance (8-35) |
| 13 | Recombinant protein | Wild-type substance (1-81)-Shortened linker-Fc fusion |
| 14 | Recombinant protein | Shortened substance (1-60)-Shortened linker-Fc fusion |
| 15 | Recombinant protein | Shortened substance (1-47)-Shortened linker-Fc fusion |
| 16 | Synthetic peptide | Shortened substance (8-35) amino acid Ala substituted (8) |
| 17 | Synthetic peptide | Shortened substance (8-35) amino acid Ala substituted (9) |
| 18 | Synthetic peptide | Shortened substance (8-35) amino acid Ala substituted (10) |
| 19 | Synthetic peptide | Shortened substance (8-35) amino acid Ala substituted (11) |
| 20 | Synthetic peptide | Shortened substance (8-35) amino acid Ala substituted (13) |
| 21 | Synthetic peptide | Shortened substance (8-35) amino acid Ala substituted (14) |
| 22 | Synthetic peptide | Shortened substance (8-35) amino acid Ala substituted (15) |
| 23 | Synthetic peptide | Shortened substance (8-35) amino acid Ala substituted (16) |
| 24 | Synthetic peptide | Shortened substance (8-35) amino acid Ala substituted (17) |
| 25 | Synthetic peptide | Shortened substance (8-35) amino acid Ala substituted (19) |
| 26 | Synthetic peptide | Shortened substance (8-35) amino acid Ala substituted (20) |
| 27 | Synthetic peptide | Shortened substance (8-35) amino acid Ala substituted (22) |
| 28 | Synthetic peptide | Shortened substance (8-35) amino acid Ala substituted (23) |
| 29 | Synthetic peptide | Shortened substance (8-35) amino acid Ala substituted (24) |
| 30 | Synthetic peptide | Shortened substance (8-35) amino acid Ala substituted (25) |
| 31 | Synthetic peptide | Shortened substance (8-35) amino acid Ala substituted (26) |
| 32 | Synthetic peptide | Shortened substance (8-35) amino acid Ala substituted (27) |
| 33 | Synthetic peptide | Shortened substance (8-35) amino acid Ala substituted (29) |
| 34 | Synthetic peptide | Shortened substance (8-35) amino acid Ala substituted (30) |
| 35 | Synthetic peptide | Shortened substance (8-35) amino acid Ala substituted (31) |
| 36 | Synthetic peptide | Shortened substance (8-35) amino acid Ala substituted (32) |
| 37 | Synthetic peptide | Shortened substance (8-35) amino acid Ala substituted (33) |
| 38 | Synthetic peptide | Shortened substance (8-35) amino acid Ala substituted (34) |
| 39 | Synthetic peptide | Shortened substance (8-35) amino acid Ala substituted (35) |
| 40 | - | Shortened linker |
| 41 | - | Repeat-type linker |
| 42 | | Fc sequence (mutant modified) |
| 43 | - | Fc sequence |
| 44 | - | GLP1 sequence |
| 45 | - | GLP1 linker |
| 46 | - | Shortened amino acid modified substance (1-60; G20S, F34M mutations introduced) |

**[Table 2]**

| SEQ ID NO: | Sequence |
|---|---|
| 1 | GSDQSENVDRGAGSIREAGGAFGKREQAEEERYFRAQSREQLAALKKHHEEEIVHHKKEIGGGGSEPKSCD KTHTCPPCPAPEAAGGPSVFLFPPKPKDTLMISRTPEVTCVVVDVSHEDPEVKFNWYVDGVEVHNAKTKPR EEQYNSTYRVVSVLTVLHQDWLNGKEYKCKVSNKALPAPIEKTISKAKGQPREPQVYTLPPSRDELTKNQV SLTCLVKGFYPSDIAVEWESNGQPENNYKTTPPVLDSDGSFFLYSKLTVDKSRWQQGNVFSCSVMHEALHN HYTQKSLSLSPGK |
| 2 | GSDQSENVDRGAGSIREAGGAFGKREQAEEERYFRAQSREQLAALKKHHEEEIVHHKKEIGGGGSGGGGSG GGGSEPKSCDKTHTCPPCPAPEAAGGPSVFLFPPKPKDTLMISRTPEVTCVVVDVSHEDPEVKFNWYVDGV EVHNAKTKPREEQYNSTYRVVSVLTVLHQDWLNGKEYKCKVSNKALPAPIEKTISKAKGQPREPQVYTLPP SRDELTKNQVSLTCLVKGFYPSDIAVEWESNGQPENNYKTTPPVLDSDGSFFLYSKLTVDKSRWQQGNVFS CSVMHEALHNHYTQKSLSLSPGK |
| 3 | GSDQSENVDRGAGSIREAGSAFGKREQAEEERYMRAQSREQLAALKKHHEEEIVHHKKEIGGGGSGGGGSG GGGSEPKSCDKTHTCPPCPAPEAAGGPSVFLFPPKPKDTLMISRTPEVTCVVVDVSHEDPEVKFNWYVDGV EVHNAKTKPREEQYNSTYRVVSVLTVLHQDWLNGKEYKCKVSNKALPAPIEKTISKAKGQPREPQVYTLPP SRDELTKNQVSLTCLVKGFYPSDIAVEWESNGQPENNYKTTPPVLDSDGSFFLYSKLTVDKSRWQQGNVFS |
| | CSVMHEALHNHYTQKSLSLSPGK |
| 4 | SPILGYWKIKGLVQPTRLLLEYLEEKYEEHLYERDEGDKWRNKKFELGLEFPNLPYYIDGDVKLTQSMAII RYIADKHNMLGGCPKERAEISMLEGAVLDIRYGVSRIAYSKDFETLKVDFLSKLPEMLKMFEDRLCHKTYL NGDHVTHPDFMLYDALDVVLYMDPMCLDAFPKLVCFKKRIEAIPQIDKYLKSSKYIAWPLQGWQATFGGGD HPPKSDLVPRGSGSDQSENVDRGAGSIREAGGAFGKREQAEEERYFRAQSREQLAALKKHHEEEIVHHKKE IERLQKEIERHKQKIKMLKHDD |
| 5 | MGSHHHHHHSSGLVPRGSHMGSDQSENVDRGAGSIREAGGAFGKREQAEEERYFRAQSREQLAALKKHHEE EIVHHKKEIERLQKEIERHKQKIKMLKHDD |
| 6 | SPILGYWKIKGLVQPTRLLLEYLEEKYEEHLYERDEGDKWRNKKFELGLEFPNLPYYIDGDVKLTQSMAII RYIADKHNMLGGCPKERAEISMLEGAVLDIRYGVSRIAYSKDFETLKVDFLSKLPEMLKMFEDRLCHKTYL NGDHVTHPDFMLYDALDVVLYMDPMCLDAFPKLVCFKKRIEAIPQIDKYLKSSKYIAWPLQGWQATFGGGD HPPKSDLVPRGSGSDQSENVDRGAGSIREAGGAFGKREQAEEERYFRAQSREQLAALKKHHEEEIVHHKKE I |
| 7 | GSDQSENVDRGAGSIREAGGAFGKREQAEEERYFRAQSREQLAALKKHHEEEIVHHKKEIGGGGSGGGGSG GGGSHGEGTFTSDVSSYLEEQAAKEFIAWLVKGGGGGGGSGGGGSGGGGSAESKYGPPCPPCPAPEFLGGP SVFLFPPKPKDTLMISRTPEVTCVVVDVSQEDPEVQFNWYVDGVEVHNAKTKPREEQFNSTYRVVSVLTVL HQDWLNGKEYKCKVSNKGLPSSIEKTISKAKGQPREPQVYTLPPSQEEMTKNQVSLTCLVKGFYPSDIAVE WESNGQPENNYKTTPPVLDSDGSFFLYSRLTVDKSRWQEGNVFSCSVMHEALHNHYTQKSLSLSLGK |
| 8 | GSDQSENVDRGAGSIREAGGAFGKREQAEEERYFRAQSREQLAALKKHHEEEIVHHKKEIERLQKEIERHK |
| | QKIKMLKHDD |
| 9 | GSDQSENVDRGAGSIREAGGAFGKREQAEEERYFRAQSREQLAALKKHHEEEIVHHKKEI |
| 10 | GSDQSENVDRGAGSIREAGGAFGKREQAEEERYFRAQSREQLAALKK |
| 11 | RGAGSIREAGGAFGKREQAEEERYFRAQSREQLAALKKHK |
| 12 | VDRGAGSIREAGGAFGKREQAEEERYFR |
| 13 | GSDQSENVDRGAGSIREAGGAFGKREQAEEERYFRAQSREQLAALKKHHEEEIVHHKKEIERLQKEIERHK QKIKMLKHDDGGGGSEPKSQDKTHTCPPCPAPELLGGPSVFLFPPKPKDTLMISRTPEVTCVVVDVSHEDP EVKFNWYVDGVEVHNAKTKPREEQYNSTYRVVSVLTVLHQDWLNGKEYKCKVSNKALPAPIEKTISKAKGQ PREPQVYTLPPSRDELTKNQVSLTCLVKGFYPSDIAVEWESNGQPENNYKTTPPVLDSDGSFFLYSKLTVD KSRWQQGNVFSCSVMHEALHNHYTQKSLSLSPG |
| 14 | GSDQSENVDRGAGSIREAGGAFGKREQAEEERYFRAQSREQLAALKKHHEEEIVHHKKEIGGGGSEPKSQD KTHTCPPCPAPELLGGPSVFLFPPKPKDTLMISRTPEVTCVVVDVSHEDPEVKFNWYVDGVEVHNAKTKPR EEQYNSTYRVVSVLTVLHQDWLNGKEYKCKVSNKALPAPIEKTISKAKGQPREPQVYTLPPSRDELTKNQV SLTCLVKGFYPSDIAVEWESNGQPENNYKTTPPVLDSDGSFFLYSKLTVDKSRWQQGNVFSCSVMHEALHN HYTQKSLSLSPG |
| 15 | GSDQSENVDRGAGSIREAGGAFGKREQAEEERYFRAQSREQLAALKKGGGGSEPKSQDKTHTCPPCPAPEL LGGPSVFLFPPKPKDTLMISRTPEVTCVVVDVSHEDPEVKFNWYVDGVEVHNAKTKPREEQYNSTYRVVSV LTVLHQDWLNGKEYKCKVSNKALPAPIEKTISKAKGQPREPQVYTLPPSRDELTKNQVSLTCLVKGFYPSD IAVEWESNGQPENNYKTTPPVLDSDGSFFLYSKLTVDKSRWQQGNVFSCSVMHEALHNHYTQKSLSLSPG |
| 16 | ADRGAGSIREAGGAFGKREQAEEERYFR |
| 17 | VARGAGSIREAGGAFGKREQAEEERYFR |
| 18 | VDAGAGSIREAGGAFGKREQAEEERYFR |
| 19 | VDRAAGSIREAGGAFGKREQAEEERYFR |
| 20 | VDRGAASIREAGGAFGKREQAEEERYFR |
| 21 | VDRGAGAIREAGGAFGKREQAEEERYFR |
| 22 | VDRGAGSAREAGGAFGKREQAEEERYFR |
| 23 | VDRGAGSIAEAGGAFGKREQAEEERYFR |
| 24 | VDRGAGSIRAAGGAFGKREQAEEERYFR |
| 25 | VDRGAGSIREAAGAFGKREQAEEERYFR |
| 26 | VDRGAGSIREAGAAFGKREQAEEERYFR |
| 27 | VDRGAGSIREAGGAAGKREQAEEERYFR |
| 28 | VDRGAGSIREAGGAFAKREQAEEERYFR |
| 29 | VDRGAGSIREAGGAFGAREQAEEERYFR |
| 30 | VDRGAGSIREAGGAFGKAEQAEEERYFR |
| 31 | VDRGAGSIREAGGAFGKRAQAEEERYFR |
| 32 | VDRGAGSIREAGGAFGKREAAEEERYFR |
| 33 | VDRGAGSIREAGGAFGKREQAAEERYFR |
| 34 | VDRGAGSIREAGGAFGKREQAEAERYFR |
| 35 | VDRGAGSIREAGGAFGKREQAEEARYFR |
| 36 | VDRGAGSIREAGGAFGKREQAEEEAYFR |
| 37 | VDRGAGSIREAGGAFGKREQAEEERAFR |
| 38 | VDRGAGSIREAGGAFGKREQAEEERYAR |
| 39 | VDRGAGSIREAGGAFGKREQAEEERYFA |
| 40 | GGGGS |
| 41 | GGGGSGGGGSGGGGS |
| 42 | EPKSCDKTHTCPPCPAPEAAGGPSVFLFPPKPKDTLMISRTPEVTCVVVDVSHEDPEVKFNWYVDGVEVHN AKTKPREEQYNSTYRVVSVLTVLHQDWLNGKEYKCKVSNKALPAPIEKTISKAKGQPREPQVYTLPPSRDE LTKNQVSLTCLVKGFYPSDIAVEWESNGQPENNYKTTPPVLDSDGSFFLYSKLTVDKSRWQQGNVFSCSVM HEALHNHYTQKSLSLSPGK |
| 43 | ESKYGPPCPPCPAPEFLGGPSVFLFPPKPKDTLMISRTPEVTCVVVDVSQEDPEVQFNWYVDGVEVHNAKT KPREEQFNSTYRVVSVLTVLHQDWLNGKEYKCKVSNKGLPSSIEKTISKAKGQPREPQVYTLPPSQEEMTK NQVSLTCLVKGFYPSDIAVEWESNGQPENNYKTTPPVLDSDGSFFLYSRLTVDKSRWQEGNVFSCSVMHEA LHNHYTQKSLSLSLGK |
| 44 | HGEGTFTSDVSSYLEEQAAKEFIAWLVKGGG |
| 45 | GGGGSGGGGSGGGGSA |
| 46 | GSDQSENVDRGAGSIREAGSAFGKREQAEEERYMRAQSREQLAALKKHHEEEIVHHKKEI |

### Example 2: Evaluation of improvement of stability in a biological system of fusion proteins

Whether the fusion proteins produced in Example 1 were stably present in vivo, they were evaluated as follows. For evaluation, ten-week-old C57BL/6 mice were used, and the mice were reared in a specialized animal facility which provided a normal diet and a 12/12 hour night/day cycle and then used for experimentation.

### 2-1. Confirmation of half-life

The synthetic peptide (SEQ ID NO: 8) and fusion protein (SEQ ID NO: 13) were each injected into the mice at a concentration of 2.5 mg/kg, and blood was collected at 0, 0.5, 2, 4, 24 hours after injection. The collected blood was centrifuged and the obtained plasma was analyzed. At this time, in order to perform effective quantification of the substances, each substance was conjugated with Dylight 680 Near-Infrared dye (NIR) in advance prior to injection, and the finally collected blood samples were examined by observing fluorescent signals with a SpectraMax i3 microplate reader device. As a result, the synthetic peptide (SEQ ID NO: 8) showed a half-life of 1.6 hours, whereas the fusion protein (SEQ ID NO: 13) showed a half-life of 18.9 hours, confirming that the half-life was increased approximately 11.8 times (Table 3). These results indicate that the fusion protein has higher stability in a biological system.

**[Table 3]**

| Test **substance** | **Half-life** |
|---|---|
| Synthetic peptide (SEQ ID NO: 8) | 1.6 hours |
| Fusion protein (SEQ ID NO: 13) | 18.9 hours |

The half-life of the fusion protein was further measured by ELISA (Enzyme-Linked Immunosorbent Assay). Each test substance was subcutaneously injected to the C57BL6/J mice at a concentration of 5 mg/kg, and then blood was separated by time.

The separated blood was added to a 96-well plate coated with a capture antibody, and each well was washed with PBS (phosphate buffered saline) 3 times. A detection solution was added to each well and the wavelengths value thereof were read at 450 nm and analyzed. As a result of the analysis, it was confirmed that the half-lives of the fusion proteins (SEQ ID NO: 1, SEQ ID NO: 2 and SEQ ID NO: 3) were increased to 39.6, 45.6, and 74.7 hours, respectively, compared with the results (Table 3) of SEQ ID NO: 8 and SEQ ID NO: 13 using the ATPIF1 full-length sequence (Table 4).

**[Table 4]**

| **Substance** | **Half-life** |
|---|---|
| Fusion protein (sequence 1) | 39.6 hours |
| Fusion protein (sequence 2) | 45.6 hours |
| Fusion protein (sequence 3) | 74.7 hours |

Such an increase in half-life was further confirmed by measuring plasma protein binding rates (Plasma protein binding test, PPB). When bound to plasma proteins (for example, albumin), they are relatively protected from proteolytic enzymes or their release was delayed, thereby increasing circulation time in blood, and also, increasing the half-life.

Each of the plasma of the mice and the fusion protein (SEQ ID NO: 13), and or His tag-wild-type substance(1-81) (SEQ ID NO: 5) was incubated at a concentration of 5 uM at 37°C for 4 hours. After that, they were centrifuged with a 100 kD filter to obtain fractions of the substance bound to the plasma proteins or fractions of the unbound substance. Each of the obtained fractions was evaluated by western blot. As a result, the fusion protein (SEQ ID NO: 13) exhibited a binding rate with the plasma proteins of 74%, whereas the binding rate of the His tag-wild-type substance (1-81) (SEQ ID NO: 5) was only 5%, and thus, it could be found that the fusion protein of the present invention showed higher in vivo stability (FIG. 1).

### Example 3: Evaluation of in vitro activity of fusion proteins

In order to verify whether the fusion proteins produced in the present invention function effectively like native proteins, the substance activity was compared with the control group in a cell system.

### 3-1. Muscle cells

In order to evaluate functions related to muscle loss diseases, each test substance was treated to the undifferentiated C2C12 mouse skeletal muscle cell line (mouse myoblast cell line) or the cell line in which differentiation into myotubes was completed. Differentiation of the cell line into myotubes was performed by conducting addition of 2% horse serum to culture medium and replacement with new medium every two days for a total of 4 days.

To the undifferentiated or differentiated cell lines, the test substances, fusion proteins (SEQ ID NOs: 1, 2, 3, 7, 14 and 15) were treated at a concentration of 100 nM (Control: PBS treated group) for 2 hours. Thereafter, the cells were immediately washed once and the cells were lysed with RIPA buffer (Radioimmunoprecipitation assay buffer). The activity of Akt and S6 (phosphorylation, p-Akt/p-S6) was observed by western blot. Akt phosphorylation is a well-known marker of activation of cell signaling pathways, and is an indicator of beneficial effects on muscle cells such as promotion of glucose metabolism and promotion of muscle biosynthesis. Similarly, S6 phosphorylation is a well-known marker of mTOR activity, and the activity of mTOR is an indicator showing muscle biosynthesis and various cell signaling pathways.

As a result of confirming the activity of Akt and S6 in the differentiated cell line, it was confirmed that the fusion proteins (SEQ ID NOs: 1, 2, 3, 7, 14 and 15) exhibited significantly higher activity than the control and His tag-wild-type substance (1-81) (SEQ ID NO: 5) (FIG. 2 and Tables 5, 6).

**[Table 5]**

| Substance | Activity (% increase compared to control) | | | |
|---|---|---|---|---|
| | Undifferentiated | | Differentiated | |
| | Akt | S6 | Akt | S6 |
| SEQ ID NO: 5 | 165% | 207% | 248% | 262% |
| SEQ ID NO: 2 | 269% | 257% | 360% | 469% |
| SEQ ID NO: 3 | 421% | 302% | 463% | 587% |
| SEQ ID NO: 7 | 377% | 513% | 411% | 446% |
| SEQ ID NO: 1 | 314% | 294% | 231% | 486% |

**[Table 6]**

| Substance | Activity (% increase compared to control) | |
|---|---|---|
| | Akt | S6 |
| Recombinant protein (SEQ ID NO: 5) control group | 254% | 217% |
| Fusion protein (SEQ ID NO: 14) experimental group | 601% | 247% |
| Fusion protein (SEQ ID NO: 15) experimental group | 548% | 232% |

### 3-2. Cancer cells

Next, the test substances, the fusion proteins (SEQ ID NO: 1, 2, 3, 7, 14, 15) and shortened sequences (SEQ ID NOs: 9, 10, 11, 12) were treated to MDA-MB-231 triple-negative breast cancer cell line at a concentration of 1 µM (Control: PBS) for 48 hours. Thereafter, the cells were washed with PBS once, and the cells were suspended in serum-free buffer. The MTT (3-[4,5-dimethylthiazol-2-yl]-2,5-diphenyltetrazolium bromide) substance was added thereto at 0.5 mg/ml and incubated at 37°C for 2 hours. Then, they were washed with PBS once, and the cells were lysed with DMSO for 15 minutes. The solution was transferred to a new plate and the absorbance was measured at 590 nm using a microplate reader.

As a result of the measurement, it could be found that the cell viability was significantly low, when the fusion proteins (SEQ ID NOs: 1, 2, 3, 7, 14, 15) or shortened sequences (SEQ ID NOs: 9, 10, 11, 12) were treated, compared with the Control, GST tag-wild-type substance (1-81) (SEQ ID NO: 4) and His tag-wild-type substance (1-81) (SEQ ID NO: 5) (FIG. 3). These results indicate that the anticancer effect was enhanced, even though the wild-type substance (1-81) (SEQ ID NO: 4, 5) were fused with other sequence or only partial sequence was used.

In addition, in order to examine generation of reactive oxygen that plays an important role in cancer cell death, each test substance was treated to the MDA-MB-231 cell line at a concentration of 1 µ M (Control: PBS) for 24 hours. Then, the cells were stained with DCF-FA and their fluorescence values were observed and compared at 485/535 nm.

As a result, it was confirmed that reactive oxygen was also generated higher in the group treated with the fusion proteins (SEQ ID NOs: 1, 2, 3, 7, 13, 14, 15) and shortened sequences (sequences 9, 10, 11), compared with the Control and His tag-wild-type substance (SEQ ID NO: 5) (FIG. 4).

### 3-3. Fat cells

To observe the functionality of the fusion proteins related to lipid metabolism, the 3T3-L1 mouse pre-adipocyte cell line was used as the cell line itself (undifferentiated) or by differentiating into adipocytes. Differentiation into the adipocytes was performed by inducing primary differentiation for the 3T3-L1 cells in a medium containing DMEM, 10% FBS, 10 ug/ml insulin, 0.5 mM IBMX (3-Isobutyl-1-methylxanthine), and 1 uM dexamethasone for 2 days, and inducing secondary differentiation with a medium containing DMEM, 10% FBS, and 10 ug/ml insulin for the remaining 4 days. The adipocytes in which differentiation was finally completed were treated with the test substances at a concentration of 100 nM for 30 hours (Control: PBS) and then used for each test.

UCP-1 (uncoupling protein 1) is a representative biomarker related to thermogenesis, indicating browning and energy metabolism activation of adipocytes. In order to observe changes in expression of UCP-1 mRNA and protein by treatment of the fusion proteins, mRNA and protein were isolated from the cells treated with each test substance and RT-PCR and western blot were performed. As a result of performing, it was confirmed that the mRNA level of UCP-1 was significantly increased by treatment of the fusion protein (SEQ ID NO: 13) (FIG. 5). These results indicate that the fusion protein (SEQ ID NO: 13) has a function in regulating energy of adipocytes.

In addition, lipid droplets of adipocytes were stained with BODIPY 493/503 dye sample, and then confocal image scanning was performed to analyze the size and diameter of the lipid droplets. As a result, it could be found that the lipid droplet size, total lipid droplet area, and maximum lipid droplet diameter per cell were reduced at a significant level by treatment of the fusion proteins (SEQ ID NOs: 8, 13) (FIG. 6).

Next, changes in cAMP (cyclic AMP), a signaling mediator contributing to lipolysis and energy metabolism were evaluated in the undifferentiated adipocytes. The undifferentiated cells were treated with the test substances at a concentration of 100 nM (Control: PBS) with 0.5 mM IBMX for 30 minutes. Thereafter, the level of cAMP was evaluated by the hTRF (Homogeneous Time Resolved Fluorescence) method (using cAMP Gs dynamic kit from Cisbio). As a result of the measurement, it was confirmed that the level of cAMP was higher in the groups treated with the fusion proteins (SEQ ID NOs: 1, 2, 3, 7, 13, 14, 15) and shortened sequences (sequences 9, 10, 11) compared with the Control and His tag-wild-type substance (1-81) (SEQ ID NO: 5) (FIG. 7).

### 3-4. Neuron cells

The neuroprotective functionality of the fusion proteins was confirmed as follows.

The SH-SY5Y dopaminergic neuron cell line was treated with 1 µ M of a toxic substance, rotenone and 100 nM of each test substance simultaneously. After 24 hours, the MTT cell viability or the effect of reducing reactive oxygen was observed.

As a result of the observation, it could be found that the cell viability was significantly increased (FGI. 8) and the generation of reactive oxygen was significantly inhibited (FIG. 9), in the groups treated with the fusion proteins (SEQ ID NOs: 1, 2, 3, 7) and shortened sequences (SEQ ID NOs: 9, 10, 11) compared with Control (group treated with rotenone alone) under the toxic environment induced by rotenone; In FIG. 8 and FIG. 9, "Control" described on the X-axis indicates treatment only with the test substance, and "+Rotenone" indicates treatment with rotenone and the test substance together.

### 3-5. Hair growth and hair loss-related functions

Whether the test substances of the present invention has effects on preventing hair loss or promoting hair growth was evaluated as follows. Specifically, it was observed whether they have efficacy of improving loss of follicular cells in scalp tissue which is a cause of hair loss, or hair growth due to an increase in hair follicles, by using dermal papilla cells and evaluating cell proliferation of follicular cells.

The follicular cells were treated with each test substance 100 nM (control: PBS) for 24 hours, and the degree of cell proliferation was evaluated by MTT assay. As a result, it could be found that the cell viability was significantly increased in the groups treated with the His tag-wild-type substance (1-81) (SEQ ID NO: 5), and the fusion proteins (SEQ ID NOs: 13, 15) compared with the Control. In particular, the fusion protein-treated groups exhibited an improved effect of promoting cell proliferation compared with the group treated with the His tag-wild-type substance (1-81), thereby demonstrating the effect of treating hair loss (FIG. 10).

### 3-6. Evaluation of binding ability

In order to evaluate substitutable residues within a sequence, an SPR (surface plasma resonance) test was conducted. Specifically, the ATP5B protein was conjugated to a CM5 chip by amine-conjugation, and then the contact of each test substance was observed under the condition of a flow rate of 50 µ l/min, and the refractive index was measured. Thereafter, by converting each binding unit, the binding ability was compared and evaluated. For the sequences of SEQ ID NOs: 17-40, the contribution of the amino acid residue affecting the binding ability was evaluated, by substituting each amino acid residue in the shortened sequence of SEQ ID NO: 16 with alanine, an inactive amino acid. A decrease of 10% or more in binding ability was considered to indicate a negative effect of the substitution.

As a result of evaluation, when the degree of binding of each sequence (SEQ ID NO: 16-39) compared with the control shortened sequence (SEQ ID NO: 12) was observed, the efficacy of the amino acid substitution of other residues, except for the sequences of SEQ ID NOs: 25, 26 and 32 of which the binding ability was reduced by 10% or more compared with SEQ ID NO: 16, was confirmed (Table 7).

**[Table 7]**

| Sequence | Binding (%) | S.D | CV (%) |
|---|---|---|---|
| 12 | 100.0 | 0.0 | 0.0 |
| 16 | 101.4 | 6.4 | 6.3 |
| 17 | 91.6 | 12.0 | 13.1 |
| 18 | 104.2 | 12.7 | 12.2 |
| 19 | 100.0 | 0.0 | 0.0 |
| 20 | 98.5 | 13.4 | 13.6 |
| 21 | 98.8 | 2.1 | 2.2 |
| 22 | 99.3 | 4.8 | 4.9 |
| 23 | 97.0 | 2.9 | 3.0 |
| 24 | 168.7 | 15.5 | 9.2 |
| 25 | 89.6 | 6.3 | 7.0 |
| 26 | 68.0 | 21.1 | 31.0 |
| 27 | 94.0 | 21.9 | 23.3 |
| 28 | 102.3 | 2.0 | 1.9 |
| 29 | 100.5 | 11.8 | 11.8 |
| 30 | 85.2 | 3.3 | 3.9 |
| 31 | 94.1 | 10.2 | 10.9 |
| 32 | 89.5 | 11.2 | 12.5 |
| 33 | 96.7 | 3.1 | 3.3 |
| 34 | 94.0 | 7.8 | 8.3 |
| 35 | 157.9 | 21.2 | 13.4 |
| 36 | 426.0 | 156.8 | 36.8 |
| 37 | 145.8 | 18.3 | 12.6 |
| 38 | 92.1 | 6.1 | 6.6 |
| 39 | 109.6 | 43.2 | 39.4 |

These results indicate that the structure of the peptide is normally maintained, and the in vivo function of the peptide can also be maintained, even when certain amino acid sequences are substituted with alanine.

### Example 4: Evaluation of in vivo activity of fusion proteins

In order to verify whether the fusion proteins prepared can exert effective functions, the activity of the substance in a biological system using a mouse model was evaluated by comparing the glucose metabolism processing ability with the control group.

### Example 4-1: Efficacy evaluation against muscle diseases - Mouse disease model

### [Sciatic nerve transection model]

The effects of the test substances on muscle diseases accompanied by muscle loss were evaluated using a sciatic nerve transection mouse model.

In the sciatic nerve transection mouse model, muscle atrophy was induced as follows. The thigh muscles of C57BL/6 male mice were transversely incised to expose sciatic nerves, and then the sciatic nerves were transected to weaken the nerves in the muscle tissue and induce muscle atrophy. The incised site was sutured, and a recovery period of one week was provided after the surgery.

To each mouse after the recovery period, the test substance (Control-PBS; and fusion protein) assigned to each group was subcutaneously injected at a concentration of 5.0 mg/kg 6 times a week for 9 weeks (Control: n=8, No.13: n=9, No.14: n=9, No.15: n=8).

The effects of improving muscle loss were measured with an X-ray body composition analyzer (DXA assay) in the fourth week of test substance administration, and the effects of improving muscle function was compared by measuring grip strength.

As a result, it was confirmed that muscle atrophy was successfully induced in the surgical site muscles, such that the muscle mass of the corresponding site was reduced in the control group upon the body composition analysis, whereas the muscle mass was significantly increased in the groups administered with the fusion proteins (sequences 13, 14, 15) (FIG. 11A). Similarly, also in the grip strength test, higher performance was observed in the fusion protein-administered groups compared with the control group, thereby observing that the muscle function was improved (FIG. 11B).

### [Duchenne muscular dystrophy (DMD)]

The effects of the test substances on muscle diseases accompanied by muscle loss were evaluated in a Duchenne muscular dystrophy mouse model (C57BL/10ScSn-Dmdmdx/J) causing progressive muscle degeneration by genetic modification.

The mouse model was divided into a Control and an experimental group. PBS to the Control, and the fusion protein (SEQ ID NO: 2) to the experimental group were subcutaneously injected at a concentration of 5 mg/kg 6 times a week for 3 weeks. After 3 weeks, it was confirmed whether the fusion protein (SEQ ID NO: 2) improved the decline in muscle function caused by muscle degeneration by conducting a grip strength test (Control: n=9, No.2: n=8).

As a result of the confirmation, it was confirmed that the grip strength was significantly improved in the group administered with the fusion protein compared with the Control, thereby demonstrating the effects of improving muscle function of the fusion protein (FIG. 12).

### Example 4-2: Cancer disease evaluation - Mouse disease model

MDA-MB-231 cells were injected into immune-deficient BALB/c nude mice, and tumors were allowed to form for one week to establish a xenograft tumor model. The mice in which tumors were formed were randomly divided into a control group and an experimental group (Control: n=8, No.14: n=7), and PBS to the control group, and the fusion protein (SEQ ID NO: 14) to the experimental group were subcutaneously injected at a dose of 7.5 mg/kg 6 times a week for a total of 6 weeks. As a result of comparing the final tumor size at the time of end of six weeks, a significant effect of inhibiting tumor growth could be confirmed in the fusion protein-administered group (FIG. 13). These results show that the fusion protein of the present invention exerts an anticancer effect in a biological system well.

### Example 4-3: Evaluation of efficacy against obesity and sarcopenic obesity diseases - Mouse disease model

### [Diet-induced obesity-induced model]

In order to confirm the efficacy of the fusion protein against diseases according to obesity and further, sarcopenic obesity, disease induction was carried out in mice through diet-induced obesity. As an animal model, C57BL/6N was used, and was used after an acclimation period by providing normal diet for 1 week, and the housing conditions were maintained at 18~24°C, and 50-60% humidity, and ad libitum feeding with free access to feed and water was carried out during both the acclimation period and experimental period. In the mice after the acclimation period for 1 week, obesity was induced by a high-fat diet (Research Diets, Inc. D12492) to induce disease.

### [Diet-induced obesity-induced model study I]

The mice in which obesity induction was completed were divided into a Control and an experimental group (Control: n=8, No.1: n=6, No.2: n=6), and PBS to the control group, and the fusion protein (SEQ ID NO: 1, 2) to the experimental group were subcutaneously injected at a dose 5 mg/kg 6 times a week for 10 weeks and the efficacy thereof was observed.

As a result of comparing final body weights, it could be confirmed that the body weight was reduced at a significant level in the fusion protein-administered group compared with the Control (FIG. 14A), and it could be confirmed that the blood sugar was also reduced (FIG. 14B).

Compared with the Control, in the fusion protein-administered group, the size of lipid droplets in liver tissue was also significantly reduced (FIG. 14C), and a significant decrease in fat tissue mass was shown when the body composition was analyzed by DXA assay at the intermediate time point (time after 3 weeks of administration) (FIG. 14D), and the muscle mass was maintained (FIG. 14E). Through these results, in an obese pathological state, not only the anti-obesity effect but also improvements of glucose metabolism and lipid metabolism, and body composition by the fusion protein were observed.

### [Diet-induced obesity-induced model study II]

While obesity induction was in progress, it was confirmed whether the fusion protein prevents obesity and suppresses muscle loss induced by obesity. During providing a high-fat diet to the mice, the fusion protein (SEQ ID NO: 14) was subcutaneously injected at a dose of 5 mg/kg 8 times for a total of 6 weeks, and the efficacy thereof was observed (Control: n=11, No.14: n=9).

As a result of comparing the final body weights, it was confirmed that compared with the Control, in the fusion protein-administered group, the increase in body weight was effectively inhibited due to development of obesity at a significant level (34.61 ± 2.33 vs 32.99 ± 2.55 g; Control vs fusion protein-administered group).

As a result of confirming the absolute value of muscle mass by DXA assay, it was relatively increased in the fusion protein-administered group, compared with the Control (18.57 ± 1.26 vs 20.46 ± 2.71 g; Control vs fusion protein-administered group), and a significant change in body composition (a decrease in fat tissue ratio/ an increase in muscle tissue ratio) (FIGs. 15A, B) could be confirmed. Furthermore, as a result of carrying out a grip strength test at the 5th week after administration of the test substance, it was confirmed that muscle function was also improved (FIG. 15C).

### [Diet-induced obesity-induced model study III]

The mice in which obesity was induced were divided into a Control and an experimental group (Control: n=8, No.3: n=7), and PBS to the Control and the fusion protein (SEQ ID NO: 3) to the experimental group were subcutaneously injected at a dose of 5 mg/kg 6 times a week for 2 weeks, and the efficacy thereof was observed.

As a result of comparing the final body weighs, it could be confirmed that the body weight was reduced at a significant level in the fusion protein-administered group, compared with the Control (FIG. 16A), and a significant change in body composition (a decrease in fat tissue ratio/ an increase in muscle tissue ratio) (FIGs. 16A, B) was observed, thereby confirming efficacy of the fusion protein.

### [Diet-induced obesity-induced model study IV]

The mice in which obesity introduction was completed (No.7: n=7) were subcutaneously injected with the fusion protein (SEQ ID NO: 7) at a dose of 5 mg/kg 6 times a week for 10 days, and the efficacy thereof was compared and observed before and after administration. As a result of comparing the experimental results by a paired t-test statistical analysis, it could be confirmed that the body weight was reduced at a significant level (FIG. 17A), and at the same time, a significant level of adipose tissue-specific reduction effect (a decrease in fat tissue ratio/ an increase in muscle tissue ratio) (FIGs. 17B, C) was observed, thereby confirming the efficacy thereof.

### [Leptin-deficient obesity disease model study]

In order to confirm the efficacy of the fusion protein in a genetic obesity condition which results in failure of hormonal regulation and inability to suppress appetite, by genetically inducing mutation of a leptin gene, and at the same time, to confirm the potential for combination therapy with a GLP-1 substance (Semaglutide), an ob/ob mouse model (C57BL/6J-ob/ob) was used.

Mice that had undergone a one-week acclimation period were divided into each group (Control: n=7, Semaglutide (30 nmol/kg): n=7, Semaglutide (30 nmol/kg) + No.14: n=6), and provided with a normal diet, and semaglutide or semaglutide+fusion protein (SEQ ID NO: 14) was subcutaneously injected to each experimental group at a dose of 5 mg/kg once per two days and the efficacy thereof was observed.

As a result of comparing the final body weights, it could be confirmed that the body weight was reduced at a significant level compared with the Control (FIG. 18A), and in particular, in terms of body composition, a more significant change (a decrease in fat tissue ratio/ an increase in muscle tissue ratio) (FIG. 18B, C) was also observed, in the combination therapy group, compared with semaglutide administration alone, thereby confirming the efficacy thereof. Furthermore, as a result of conducing a grip strength test, it was confirmed that muscle function was also improved (FIG. 18D).

### Example 4-4: Evaluation of fatty acid and lipid metabolic diseases - Mouse disease model

5-week-old female C57BL/6J mice were obtained and subjected to an acclimation period of 1 week, and then fatty liver induction was performed. The housing conditions of all the mice were always maintained at 18~24°C, 50-60% humidity, and ad libitum feeding was carried out during both the acclimation period and fatty liver induction period. After completing the acclimation period of 1 week, the mice were provided with a GAN diet (Gubra Amylin NASH diet) for 38 weeks to induce non-alcoholic fatty liver. The detailed composition of the product is as Table 8 below.

**[Table 8]**

| |
|---|
| 40 kcal% fat (palm oil) |
| 20 kcal% fructose |
| 2% cholesterol |

The mice in which non-alcoholic fatty liver was induced were divided into a control group (n=7) and an experimental group (n=6). PBS to the control group, and the fusion protein (SEQ ID NO: 14) to the experimental group were subcutaneously administered at a dose of 2.5 mg/kg 6 times a week, for 5 weeks. At the end of the test, the weights of adipose tissue and liver tissue were measured, and biochemical tests (OT, PT, Cholesterol, TG), and histological staining (Masson's trichrome) were performed. Masson's trichrome staining was used for analyzing the degree of collagen accumulation, which is the cause of liver fibrosis induced from fatty liver disease, and inflammatory cell infiltration, which is the cause of inflammation, since it stains collagen fibers blue, cytoplasm red, and cell nuclei dark brown.

As a result of performing, significant decreases in body weight and decreases in adipose tissue, and decreases in liver weight increase according to fatty liver induction could be confirmed in the experimental group compared with the control group. In addition, it was observed that the levels of OT (GOT, AST (aspartate aminotransferase)), PT (GPT, ALT (alanine aminotransferase)). which are detected in response to liver tissue damage, were significantly reduced, and at the same time, blood cholesterol was significantly reduced and triglyceride (TG) was reduced, in the experimental group, compared with the control group, thereby confirming a positive effect throughout lipid metabolism. Furthermore, it was confirmed that the degree of collagen accumulation was reduced in the Masson's trichrome staining (Table 9), thereby verifying the efficacy of the fusion protein against fatty liver and lipid metabolic diseases.

**[Table 9]**

| No. | Test item | Control group | Experimental group |
|---|---|---|---|
| 1 | BW (g ± SD) | 35.64 ± 3.07 | 30.53 ± 4.62 |
| 2 | Liver (g ± SD) | 1.64 ± 0.20 | 1.59 ± 0.11 |
| 3 | Epididymal Fat depot (g ± SD) | 2.62 ± 0.67 | 1.50 ± 0.72 |
| 4 | OT (U/L ± SD) | 285.66 ± 46.95 | 196.70 ± 38.38 |
| 5 | PT (U/L ± SD) | 138.46 ± 29.03 | 94.07 ± 36.03 |
| 6 | Cholesterol (mg/dl ± SD) | 128.57 ± 21.32 | 102.30 ± 16.69 |
| 7 | TG (mg/dl ± SD) | 13.14 ± 9.58 | 10.27 ± 2.80 |
| 8 | Collagen Area (% ± SD) | 7.08 ± 3.81 | 5.67 ± 3.01 |

### Example 5-5: Evaluation of glucose metabolic diseases - Mouse disease model

### [Diet-induced obesity-induced impaired glucose tolerance disease model study]

In order to secure an impaired glucose tolerance model, conditions causing glucose metabolic disorders in mice by diet-induced obesity were designed. As an animal model, 5-week-old male C57BL/6N mice were obtained and after undergoing an acclimation period by providing a normal diet for 1 week, they were used, and the housing conditions were maintained at 18~24°C, 50-60 % humidity, and ad libitum feeding with free access to feed and water was carried out during both the acclimation period and experimental period.

In order to induce impaired glucose tolerance to the mice which had undergone the acclimation period of 1 week, a high-fat diet was supplied to induce obesity. The high-fat diet was provided by preparing as Table 10 below.

**[Table 10]**

| Ingredients | HFD (g) |
|---|---|
| Corn Starch | 15 |
| Casein | 20 |
| Sucrose | 34 |
| Corn oil | 3 |
| Mineral mix | 3.5 |
| Vitamin mix | 1 |
| Cellulose | 5 |
| DL-methionine | 0.3 |
| Choline bitartrate | 0.2 |
| Lard | 17 |
| Cholesterol | 1 |
| BHT | 0.001 |
| Total | 100 |

The obesity-induced mice were divided into a control group (negative control group; n=14) and an experimental group (fusion protein SEQ ID NOs: 14, 15; No.14: n=9, No.15: n=9) and subjected to fasting for 8 hours. Thereafter, PBS to the control, and the fusion protein to the experimental group were administered at a concentration of 5.0 mg/kg, and after 1 hours, a 20% glucose solution was intraperitoneally injected. Blood was collected at each time point and analyzed using a glucometer.

As a result of the analysis, when the blood glucose variation over time of the fusion protein-administered group was quantified and compared with the control group (GLUAUC), a significant difference was shown (FIG. 19), thereby confirming the effect of improving impaired glucose tolerance by the fusion proteins.

### [Leptin receptor-deficient diabetes diseases model study I]

In order to confirm efficacy of the fusion proteins in a diabetic pathological condition in which impaired glucose tolerance by genetically inducing mutation to a leptin receptor gene, a 7-week-old db/db mouse model (C57BLKS/J-db/db) was used. The mice after a one-week acclimation period were divided into a control (n=7) and an experimental group (fusion protein SEQ ID NO: 2; n=8). The mice was provided with a normal diet, and for 4 weeks, PBS to the control, and the fusion protein to the experimental group were subcutaneously injected at a dose of 5 mg/kg 6 times a week, and the efficacy thereof was observed.

As a result of the observation, it could be found that the body composition which had been altered by damaged biological metabolism due to diabetes was improved (a decrease in fat tissue ratio/ an increase in muscle tissue ratio) by the fusion protein (Table 11). Furthermore, as a result of conducting a grip strength test, it was confirmed that muscle function was also significantly improved (FIG. 20).

**[Table 11]**

| No. | Test item | Control group | Experimental group |
|---|---|---|---|
| 1 | Fat tissue ratio (% ± SD) | 52.41 ± 3.43 | 50.10 ± 3.26 |
| 2 | Muscle tissue ratio (% ± SD) | 47.59 ± 3.43 | 49.90 ± 3.26 |

In summary, the fusion proteins of the present invention are considered to be capable of exerting more effective substance function based on the high stability in the biological system and the improved activity verified in the cell system.

### [Leptin receptor-deficient diabetes diseases model study II]

In order to confirm efficacy of the fusion proteins in a diabetic pathological condition in which impaired glucose tolerance by genetically inducing mutation to a leptin receptor gene, a 9-week-old db/db mouse model (C57BLKS/J-db/db) was used. The mice after a one-week acclimation period were divided into a control (n=9) and an experimental group (fusion protein SEQ ID NO: 2; n=7). The mice was provided with a normal diet, and for 11 weeks, PBS to the control, and the fusion protein to the experimental group were subcutaneously injected at a dose of 5 mg/kg 6 times a week, and the efficacy thereof was observed.

As a result of the observation, it was confirmed that the blood glucose level which had been altered by damaged glucose metabolism due to diabetes was significantly reduced by administration of the fusion protein, thereby confirming its efficacy of the fusion protein against glucose metabolic diseases (FIG. 21).

## Claims

1. A fusion protein consisting of an amino acid sequence selected from the group consisting of SEQ ID NOs: 8 to 10 and 46;
a linker connected to the C-terminus of the amino acid sequence; and
an Fc sequence connected to a terminus of the linker.

2. The fusion protein according to claim 1, wherein the liker is a sequence of SEQ ID NO: 40 or SEQ ID NO: 41.

3. The fusion protein according to claim 1, wherein the Fc sequence is a sequence of SEQ ID NO: 42 or SEQ ID NO: 43.

4. The fusion protein according to claim 1, wherein in the amino acid sequence selected from the group consisting of SEQ ID NOs: 8 to 10 and 46, one or more amino acids selected from the group consisting of the 8th, 9th, 10th, 11th, 13th, 14th, 15th, 16th, 17th, 22th, 23th, 24th, 25th, 26th, 29th, 30th, 31th, 32th, 33th, 34th and 31th amino acids are substituted with alanine.

5. The fusion protein according to claim 1, wherein the fusion protein consists of a sequence selected from the group consisting of SEQ ID NOs: 1 to 3, 13, 14 and 15.

6. The fusion protein according to claim 1, wherein the fusion protein consists of the following
an amino acid sequence selected from the group consisting of SEQ ID NOs: 8 to 10 and 46;
a linker connected to the C-terminus of the amino acid sequence;
a GLP-1 (Glucagon like peptide-1) sequence consisting of SEQ ID NO: 44 connected to a terminus of the linker;
a GLP-1 linker consisting of SEQ ID NO: 45 connected to a terminus of the GLP-1 sequence; and
an Fc sequence connected to a terminus of the GLP-1 linker.

7. The fusion protein according to claim 1, wherein the fusion protein consists of the sequence of SEQ ID NO: 7.

8. A pharmaceutical composition for preventing or treating cancer comprising the fusion protein according to claim 1 as an active ingredient.

9. A pharmaceutical composition for preventing or treating muscle diseases associated with muscle loss comprising the fusion protein according to claim 1 as an active ingredient.

10. A pharmaceutical composition for preventing or treating metabolic syndrome comprising the fusion protein according to claim 1 as an active ingredient.

11. A pharmaceutical composition for preventing or treating nonalcoholic fatty liver disease comprising the fusion protein according to claim 1 as an active ingredient.

12. A pharmaceutical composition for preventing hair loss or promoting hair growth comprising the fusion protein according to claim 1 as an active ingredient.

13. A pharmaceutical composition for protecting brain neuron cells comprising the fusion protein according to claim 1 as an active ingredient.
